(19)

Europäisches
Patentamt
European
Patent Office
Office européen
des brevets

(11) **EP 1 867 642 A1**

(12) **EUROPEAN PATENT APPLICATION**
published in accordance with Art. 158(3) EPC

(43) Date of publication:
 **19.12.2007 Bulletin 2007/51**

(21) Application number: **06731003.7**

(22) Date of filing: **03.04.2006**

(51) Int Cl.:
 *C07D 307/77* (2006.01)  *A61K 31/343* (2006.01)
 *A61K 31/13* (2006.01)  *A61K 31/165* (2006.01)
 *A61K 31/445* (2006.01)  *A61K 31/473* (2006.01)
 *A61K 31/55* (2006.01)  *A61K 45/00* (2006.01)
 *A61P 25/20* (2006.01)  *A61P 25/28* (2006.01)
 *A61P 43/00* (2006.01)

(86) International application number:
 **PCT/JP2006/307055**

(87) International publication number:
 **WO 2006/107027 (12.10.2006 Gazette 2006/41)**

(84) Designated Contracting States:
 **AT BE BG CH CY CZ DE DK EE ES FI FR GB GR
 HU IE IS IT LI LT LU LV MC NL PL PT RO SE SI
 SK TR**
 Designated Extension States:
 **AL BA HR MK YU**

(30) Priority: **04.04.2005 JP 2005107673**

(71) Applicant: **Takeda Pharmaceutical Company
 Limited
 Osaka-shi,
 Osaka 541-0045 (JP)**

(72) Inventors:
 • **HIRAI, Keisuke,
 TAKEDA PHARMACEUTICAL COMPANY LTD.
 Osaka-shi,
 Osaka; 5328686 (JP)**
 • **MIYAMOTO, Masaomi,
 TAKEDA PHARMACEUTICAL COMP. LTD
 Osaka-shi, Osaka (JP)**

(74) Representative: **Rickard, Timothy Mark Adrian
 Takeda Euro IP Department
 Arundel Great Court
 2 Arundel Street
 London
 WC2R 3DA (GB)**

(54) **AGENT FOR PREVENTION OR TREATMENT OF NOCTURNAL BEHAVIORAL DISORDER
 ASSOCIATED WITH DEMENTIA**

(57)  The present invention provides a prophylactic or therapeutic agent for nocturnal conduct disorders associated with dementia, comprising (S)-N-[2-(1,6,7,8-tetrahydro-2H-indeno[5,4-b]furan-8-yl)ethyl]propionamide.

EP 1 867 642 A1

**Description**

Technical Field

The present invention relates to an inhibitor for nocturnal conduct disorder associated with dementia.

Background Art

Dementia is a so-called mental deterioration, and is a condition or symptom interfered with social life and occupational function by cognitive impairment. Here, 'cognition' is a concept generalizing intellectual function such as learn, see, hear, talk, think, and the like. Patients with dementia have a core symptom such as aphasia (language disorder), apraxia (unable to execute motor activity in spite of normal motor function), agnosia (unable to recognize and identify an object in spite of normal sensory function), executive dysfunction (unable to plan and execute), and the like in addition to memory hypofunction. Further, the core symptom such as dysmnesia causes psychoneurosis such as disorder of emotion and motivation, and conduct disorder such as hallucinations, delusions, wandering, and resistance to care. Furthermore, it is reported that in the patients with dementia, a sleep disorder is occasionally developped (for example, see non-patent document 1, non-patent document 2, etc.). Since family members burdened with caring are often bothered with rather peripheral symptoms such as late-night wandering than core symptoms, development of medicines for preventing or treating conduct disorders is desired.

[patent document 1] US Patent No. 6,034,239

[non-patent document 1] Vitiello MV, Borson S. CNS Drugs. 2001; 15: 777-96, Sleep disturbances in patients with Alzheimer's disease: epidemiology, pathophysiology and treatment

[non-patent document 2] Bliwise DL Clin Cornerstone. 2004; 6: S16-28, Sleep disorders in Alzheimer's disease and other dementias

Disclosure of Invention

Problems to be Solved by the Invention

The object of the present invention is to provide a prophylactic or therapeutic agent for nocturnal conduct disorders associated with dementia.

Means of Solving the Problems

In the patients with dementia, melatonin levels are reduced (for example, see Luboshitzky R, Shen-Orr Z, Tzischichinsky O, Maldonado M, Herer P, Lavie P. Chronobiol Int. 2001; 18: 513-24, Actigraphic sleep-wake patterns and urinary 6-sulfatoxymelatonin excretion in patients with Alzheimer's disease, Mishima K, Tozawa T, Satoh K, Matsumoto Y, Hishikawa Y, Okawa M, Biol Psychiatry. 1999; 45: 417-21, Melatonin secretion rhythm disorders in patients with senile dementia of Alzheimer's type with disturbed sleep-waking, etc.). The present inventors have considered that this is the cause of conduct disorders (in particular, at night), and found out that nocturnal conduct disorders can be inhibited by administering (S)-N-[2-(1,6,7,8-tetrahydro-2H-indeno[5,4-b]furan-8-yl)ethyl]propionamide (hereinafter, sometimes referred to as compound A) which is a melatonin agonist, and finally completed the present invention.

That is, the present invention provides:

[1] A pharmaceutical composition for prevention or treatment of nocturnal conduct disorders associated with dementia, which comprises (S)-N-[2-(1,6,7,8-tetrahydro-2H-indeno[5,4-b]furan-8-yl)ethyl]propionamide;

[2] A pharmaceutical composition for prevention or treatment of dementia, which comprises (S)-N-[2-(1,6,7,8-tetrahydro-2H-indeno[5,4-b]furan-8-yl)ethyl]propionamide in combination with one or more drugs selected from an acetylcholinesterase inhibitor and a N-methyl-D-aspartic acid receptor antagonist;

[3] A pharmaceutical composition for prevention or treatment of dementia, which comprises (S)-N-[2-(1,6,7,8-tetrahydro-2H-indeno[5,4-b]furan-8-yl)ethyl]propionamide in combination with one or more drugs selected from donepezil, galantamine, rivastigmine, tacrine, and memantine;

[4] A method for preventing or treating nocturnal conduct disorders associated with dementia, which comprises administering (S)-N-[2-(1,6,7,8-tetrahydro-2H-indeno[5,4-b]furan-8-yl)ethyl]propionamide;

[5] A method for preventing or treating dementia, which comprises administering (S)-N-[2-(1,6,7,8-tetrahydro-2H-indeno[5,4-b]furan-8-yl)ethyl]propionamide in combination with one or more drugs selected from an acetylcholinesterase inhibitor and a N-methyl-D-aspartic acid receptor antagonist;

[6] A method for preventing or treating dementia, which comprises administering (S)-N-[2-(1,6,7,8-tetrahydro-2H-indeno[5,4-b]furan-8-yl)ethyl]propionamide in combination with one or more drugs selected from donepezil, galantamine, rivastigmine, tacrine, and memantine;

[7] Use of (S)-N-[2-(1,6,7,8-tetrahydro-2H-indeno[5,4-b]furan-8-yl)ethyl]propionamide for manufacturing a pharmaceutical composition for prevention or treatment of nocturnal conduct disorders associated with dementia;

[8] Use of (S)-N-[2-(1,6,7,8-tetrahydro-2H-indeno[5,4-b]furan-8-yl)ethyl]propionamide in combination with one or more drugs selected from an acetylcholinesterase inhibitor and a N-methyl-D-aspartic acid receptor antagonist for manufacturing a pharmaceutical composition for prevention or treatment of dementia; and

[9] Use of (S)-N-[2-(1,6,7,8-tetrahydro-2H-indeno[5,4-b]furan-8-yl)ethyl]propionamide in combination with one or more drugs selected from donepezil, galantamine, rivastigmine, tacrine, and memantine for manufacturing a pharmaceutical composition for prevention or treatment of dementia; and the like.

Best Mode for Carrying Out the Invention

(S)-N-[2-(1,6,7,8-Tetrahydro-2H-indeno[5,4-b]furan-8-yl)ethyl]propionamide, of which generic name is Ramelteon, and which may be hereinafter referred to as compound A, to be used in the present invention is a known therapeutic agent for sleep disorders disclosed in US 6,034,239 etc., and can be produced by a known method such as that disclosed in the reference.

Compound A has a melatonin agonistic action. Therefore, compound A can be used for preventing or treating conduct disorders associated with dementia. In particular, since compound A has a therapeutic effect on sleep disorders, it is effective for the inhibition of nocturnal conduct disorders such as late-night wandering and the like.

In addition, compound A is extremely low toxic and has no influence on learning activities, and, thus, it can be used for a prevention or treatment of dementia by combining with an antidimentia drug. The prevention or treatment of dementia includes a prevention or treatment of nocturnal conduct disorders. Further, as used herein, the dementia includes Alzheimer's disease, mild cognitive impairment, and senile dementia.

Examples of such antidimentia drug include acetylcholinesterase inhibitors such as Donepezil, Galantamine, Rivastigmine, and Tacrine, N-methyl-D-aspartic acid (NMDA) receptor antagonists such as Memantine, PPARγ agonists such as rosiglitazone, γ-secretase inhibitors such as LY-450139, 5HT1A receptor agonists having neurotrophic factor activity such as Xaliproden, 5HT4 partial agonists such as SL-650155, MAO-B inhibitor such as SR-57667, Ab aggregation inhibitors such as alzhemed, β-secretase inhibitors such as (R)-(+)-6-(4-biphenylyl)methoxy-2-[2-(N,N-dimethylamino) ethyl]tetralin hydrochloride · $1H_2O$, and the like.

These antidimentia drugs may be a free form or a pharmaceutically acceptable salt. Examples of the salts include, in case that the antidimentia drugs have an acidic functional group, inorganic salts such as alkali metal salts (e.g., sodium salt, potassium salt, etc.), alkaline earth metal salts (e.g., calcium salt, magnesium salt, barium salt, etc.), ammonium salt, and the like. In addition, in case that the antidimentia drugs have a basic functional group, examples of the salts include salts with inorganic acid such as hydrochloric acid, hydrobromic acid, nitric acid, sulfuric acid, phosphoric acid, and the like, and salts with organic acid such as acetic acid, phthalic acid, fumaric acid, oxalic acid, tartaric acid, maleic acid, citric acid, succinic acid, methanesulfonic acid, p-toluenesulfonic acid, and the like. The known antidimentia drugs exemplified here can be commercially available with ease, or can be produced according to a known method.

Among the antidimentia drugs, some have an adverse effect of sleep disorder (e.g., donepezil). Since compound A has a therapeutic effect on sleep disorders, it can preferably be used in combination with such antidimentia drugs. The antidimentia drugs include acetylcholinesterase inhibitors and N-methyl-D-aspartic acid (NMDA) receptor antagonists.

Furthermore, compound A can also be used in combination with a therapeutic agent for diseases associated with sleep disorders.

When compound A is used in combination with antidimentia drugs, examples of administration forms include (1) administration of a single preparation obtained by formulating compound A and an antidimentia drug simultaneously, (2) simultaneous administration of two preparations obtained by formulating compound A and an antidimentia drug separately, via an identical route, (3) sequential and intermittent administration of two preparations obtained by formulating compound A and an antidimentia drug separately, via an identical route, (4) simultaneous administration of two preparations obtained by formulating compound A and an antidimentia drug separately, via different routes, (5) sequential and intermittent administration of two preparations obtained by formulating compound A and an antidimentia drug separately, via different routes (e.g. administration in the order of compound A → antidimentia drug, or in the inverse order) and the like. From a viewpoint of convenience of patients, preferred is an administration of a single preparation obtained by formulating compound A and an antidimentia drug simultaneously.

The dosage of the combined drug can be appropriately selected based on a clinically used dose. In addition, the blending ratio of compound A and antidimentia drug can be appropriately selected depending on administration subject, administration route, target disease, symptom, antidimentia drug to be used and the like. Usually, the ratio may be decided based on the general dose of the antidimentia drug to be used. When the administration subject is human, for example, 0.01-100 parts by weight of the antidimentia drug is used relative to 1 part by weight of compound A.

Compound A can be safely administered orally or parenterally (e.g. topically, rectally, intravenously etc.) as it is or as a pharmaceutical composition mixed with pharmacologically acceptable carriers according to a conventional method (e.g., method described in Japanese Pharmacopoeia, etc.), such as tablets (including sugar-coated tablets, film-coated tablets), powders, granules, capsules, solutions, emulsions, suspensions, injectables, suppositories, sustained-release agents, adhesive preparations, and the like.

The content of compound A is usually about 0.01 to 100% by weight based on total weight of the composition.

The dose of compound A differs depending on an administration subject, administration route, and disease. For example, as a therapeutic agent for sleep disorders, the dosage is about 0.0005 to 2 mg/kg body weight, preferably about 0.001 to 1 mg/kg body weight, more preferably about 0.001 to 0.5 mg/kg body weight in terms of compound (I) as active ingredient for an adult. The pharmaceutical composition may be administered once to several times in divided doses per day.

[Examples]

The present invention will be described in detail through the following Preparation Examples and Experiments. However, these are just an example, and the present invention is not limited by the examples, and may be changed without departing from the scope of the present invention.

Preparation Example 1

Compound A (160 g), lactose (4064 g), and corn starch (640 g) are mixed uniformly in a fluidized bed granulation dryer, and the mixture is granulated with spraying a solution of hydroxypropyl cellulose (160 g) in water in the dryer, followed by drying in said drier. The resulting granulated material is crushed by 1.5 mmφ punching screen using a power mill apparatus to obtain uniform granules. To the uniform granules (3894 g) are added corn starch (124 g) and magnesium stearate (12.4 g), and the mixture is mixed to give granules for tableting. These granules are tableted in a weight of 130 mg per tablet with a 7.0 mmφ die using a tableting machine to prepare bare tablets. The obtained bare tablets are sprayed with a solution of hydroxypropylmethylcellulose 2910 and copolividone wherein titanium oxide and yellow ferric oxide are dispersed, in a film coating machine, to give about 25000 tablets which are film-coated tablets each containing 4 mg of compound A per tablet and having a prescription shown in Table 1.

[Table 1]

| Composition | Blending Quantity (mg) |
|---|---|
| Compound A | 4.0 |
| Lactose | 101.6 |
| Corn Starch | 20.0 |
| Hydroxypropyl Cellulose | 4.0 |
| Magnesium stearate | 0.4 |
| Bare Tablet | 130.0 |
| Hydroxypropylmethylcellulose 2910 | 3.74 |
| Copolividone | 0.75 |
| Titanium Oxide | 0.5 |
| Yellow Ferric Oxide | 0.01 |
| Total | 135.0 |

Preparation Example 2

Compound A (160 g), Donepezil (160 g), lactose (4064 g), and corn starch (640 g) are mixed uniformly in a fluidized bed granulation dryer, and the mixture is granulated with spraying a solution of hydroxypropyl cellulose (160 g) in water in the dryer, followed by drying in said drier. The resulting granulated material is crushed by 1.5 mmφ punching screen using a power mill apparatus to obtain uniform granules. To the uniform granules (3894 g) are added corn starch (124 g) and magnesium stearate (12.4 g), and the mixture is mixed to give granules for tableting. These granules are tableted using a tableting machine to prepare bare tablets. The obtained bare tablets are sprayed with a solution of hydroxypropylmethylcellulose 2910 and copolividone wherein titanium oxide and yellow ferric oxide are dispersed, in a film coating machine, to give film-coated tablets each containing 4 mg of compound A and 4 mg of Donepezil per tablet and having a prescription shown in Table 2.

[Table 2]

| Composition | Blending Quantity (mg) |
|---|---|
| Compound A | 4.0 |
| Donepezil | 4.0 |
| Lactose | 101.6 |
| Corn Starch | 20.0 |
| Hydroxypropyl Cellulose | 4.0 |
| Magnesium stearate | 0.4 |
| Bare Tablet | 134.0 |
| Hydroxypropylmethylcellulose 2910 | 3.74 |
| Copolividone | 0.75 |
| Titanium Oxide | 0.5 |
| Yellow Ferric Oxide | 0.01 |
| Total | 139.0 |

Experiment 1

1) Measurement of rotational motor activity in mouse Animals: SAMP8/Ta Slc mice (16 weeks old), and SAMR1/Ta Slc mice (16 weeks old) as control mouse were used. After breeding in a rearing room, they were used after the age of 6-month-old to 8-month-old. A sole isolation breeding was conducted during the breeding.

AB system of Neuroscience Co. was used for measurement. The mice were put in separate cages, and the turning wheel's rotation number for every minute by mouse was measured, and the data was analyzed as a motor activity for every hour. A turning wheel was equipped in the cage for measurement. The number in which the mouse rotated the turning wheel was automatically counted every minute with AB system (Neuroscience Co.), and recorded with time course as an amount of movement (motor activity).

2) Used Drug and Administration Method

Experiment was carried out according to the following schedule.

Using the total value of turning wheel's rotation number in habituation stage, each group was evenly grouped. Compound A was dissolved in water for injection, and water for injection was used as a vehicle. The solution and vehicle were administered as drinking water, respectively. The drinking water was replaced with new one every week.

Pre: Habituation period (1 week)
First week: Vehicle/Compound A 0.1-0.2 mg/kg/day
Second week: Vehicle/Compound A 0.1-0.2 mg/kg/day
Third week: Vehicle/Compound A 1-2 mg/kg/day
Fourth week: Vehicle/Compound A 1-2 mg/kg/day
Fifth week: Vehicle/Compound A 1-2 mg/kg/day

3) Data Analysis

The number of rotational movement which was automatically counted every minute by AB system (Neuroscience Co.) was tallied for every hour.

Calculation was carried out according to the following sequences.

(i) By calculating according to the following mathematical formula:

(rotational motor activity in light period of a day)/(rotational motor activity in light period of a day + rotational motor activity in dark period of a day) x 100,

motor activity % in light period was determined.

(ii) The mean value of motor activity % in light period was calculated for every week, and was made a representative value for every week.

(iii) In addition, in case that the value of Pre was made 100%, rate of change of motor activity % in light period after administration was shown as % of Pre value.

4) Statistical Analysis

Statistical analysis was performed with t-test or Paired t-test for vehicle administration group and compound A administration group of SAMP8.

5) Result

The test results were shown in Table 3.

[Table 3] Effect of Compound A on increase of motor activity in light period in SAMP8

**Motor Activity in light period, %**

|  | Pre | 1st week | 2nd week | 3rd week | 4th week | 5th week |
|---|---|---|---|---|---|---|
| **SAMR1** |  |  |  |  |  |  |
| Vehicle | 3.90 | 7.91 | 5.75 | 6.56 | 7.08 | 4.96 |
| **SAMP8** |  |  |  |  |  |  |
| Vehicle | 25.73 | 27.36 | 25.06 | 26.27 | 24.70 | 24.53 |
| Compound A | 31.29 | 30.95 | 24.58 | 22.82 * | 23.75 * | 21.18 ** |

**Motor Activity in light period, % of Pre**

|  | Pre | 1st week | 2nd week | 3rd week | 4th week | 5th week |
|---|---|---|---|---|---|---|
| **SAMP8** |  |  |  |  |  |  |
| Vehicle | 100.00 | 98.54 | 88.98 | 98.05 | 95.70 | 103.62 |
| Compound A | 100.00 | 97.67 | 79.60 | 70.25 ** | 74.77 * | 71.64 * |

Paired t test    *: $p < 0.05$,    **: $p < 0.01$

Administration dose:    1st week: Compound A 0.1-0.2 mg/kg

2nd week: Compound A 0.1-0.2 mg/kg

3rd week: Compound A 1-2 mg/kg

4th week: Compound A 1-2 mg/kg

5th week: Compound A 1-2 mg/kg

n=9

It is known that when an age-related change of circadian rhythm for spontaneous motor activity of SAMP8 mouse in 12-hour/12-hour light-dark cycle is studied in comparison with SAMR1 as control, SAMR1 shows a typical circadian rhythm which is high in dark period and low in light period like other rodents, but SAMP8 shows apparently an abnormality of rhythm, that is, shows a high spontaneous motor activity after light period. In the present experiment, such difference between SAMR1 and SAMP8 was also observed. In order to check whether administration of drinking water containing compound A has a decreasing effect on the increase of rotational motor activity in light period of SAMP8, data analysis was conducted on the basis of the ratio of motor activity in light period in a day.

Administration by free drinking of water containing compound A was started at a dose of 0.1-0.2 mg/kg/day in terms of compound A, and the data was analyzed every week, and then, the dose was appropriately escalated referring to the course of the drug efficacy. In the first and second week after commencing administration of drinking water to SAMP8, there was not observed a change of the motor activity % in light period and the % of Pre value that is a rate of change to the Pre value, compared to the vehicle administered group, and the change was considered to be within the margin of error. When the dose was increased to 1-2 mg/kg/day in terms of compound A from the third week to the fifth week, it was confirmed that the motor activity % in light period and the % of Pre value that is a rate of change to the Pre value were decreased, compared to the vehicle-administered group. It became apparent in the Paired t test that the rate of change of motor activity % in light period and % of Pre is significantly decreased in the compound A-administered group. Therefore, it was made obvious that compound A can inhibit dose-dependently the abnormal behavior in light period of SAMP8. On the other hand, it was confirmed that SAMR1 used as control mouse has a rhythm of nocturnal increase and diurnal decrease of rotational motor activity. In addition, changes of motor activity before and after administration of drinking water (dark period, light period, all day) were not observed.

Industrial Applicability

According to the present invention, there are provided a prophylactic or therapeutic agent for nocturnal problematic behaviors associated with dementia, and the like.

**Claims**

1. A pharmaceutical composition for prevention or treatment of nocturnal conduct disorders associated with dementia, which comprises (S)-N-[2-(1,6,7,8-tetrahydro-2H-indeno[5,4-b]furan-8-yl)ethyl]propionamide.

2. A pharmaceutical composition for prevention or treatment of dementia, which comprises (S)-N-[2-(1,6,7,8-tetrahydro-2H-indeno[5,4-b]furan-8-yl)ethyl]propionamide in combination with one or more drugs selected from an acetylcholinesterase inhibitor and a N-methyl-D-aspartic acid receptor antagonist.

3. A pharmaceutical composition for prevention or treatment of dementia, which comprises (S)-N-[2-(1,6,7,8-tetrahydro-2H-indeno[5,4-b]furan-8-yl)ethyl]propionamide in combination with one or more drugs selected from donepezil, galantamine, rivastigmine, tacrine, and memantine.

4. A method for preventing or treating nocturnal conduct disorders associated with dementia, which comprises administering (S)-N-[2-(1,6,7,8-tetrahydro-2H-indeno[5,4-b]furan-8-yl)ethyl]propionamide.

5. A method for preventing or treating dementia, which comprises administering (S)-N-[2-(1,6,7,8-tetrahydro-2H-indeno[5,4-b]furan-8-yl)ethyl]propionamide in combination with one or more drugs selected from an acetylcholinesterase inhibitor and a N-methyl-D-aspartic acid receptor antagonist.

6. A method for preventing or treating dementia, which comprises administering (S)-N-[2-(1,6,7,8-tetrahydro-2H-indeno[5,4-b]furan-8-yl)ethyl]propionamide in combination with one or more drugs selected from donepezil, galantamine, rivastigmine, tacrine, and memantine.

7. Use of (S)-N-[2-(1,6,7,8-tetrahydro-2H-indeno[5,4-b]furan-8-yl)ethyl]propionamide for manufacturing a pharmaceutical composition for prevention or treatment of nocturnal conduct disorders associated with dementia.

8. Use of (S)-N-[2-(1,6,7,8-tetrahydro-2H-indeno[5,4-b]furan-8-yl)ethyl]propionamide in combination with one or more drugs selected from an acetylcholinesterase inhibitor and a N-methyl-D-aspartic acid receptor antagonist for manufacturing a pharmaceutical composition for prevention or treatment of dementia.

9. Use of (S)-N-[2-(1,6,7,8-tetrahydro-2H-indeno[5,4-b]furan-8-yl)ethyl]propionamide in combination with one or more drugs selected from donepezil, galantamine, rivastigmine, tacrine, and memantine for manufacturing a pharmaceutical composition for prevention or treatment of dementia.

**INTERNATIONAL SEARCH REPORT**

| | International application No. |
|---|---|
| | PCT/JP2006/307055 |

A. CLASSIFICATION OF SUBJECT MATTER
*C07D307/77*(2006.01), *A61K31/343*(2006.01), *A61K31/13*(2006.01), *A61K31/165*
(2006.01), *A61K31/445*(2006.01), *A61K31/473*(2006.01), *A61K31/55*(2006.01),
*A61K45/00*(2006.01), *A61P25/20*(2006.01), *A61P25/28*(2006.01),
According to International Patent Classification (IPC) or to both national classification and IPC

B. FIELDS SEARCHED

Minimum documentation searched (classification system followed by classification symbols)
A61K31/13, A61K31/165, A61K31/343, A61K31/445, A61K31/473, A61K31/55,
A61K45/00, A61P25/20, A61P25/28, A61P43/00, C07D307/77

Documentation searched other than minimum documentation to the extent that such documents are included in the fields searched
Jitsuyo Shinan Koho          1922-1996   Jitsuyo Shinan Toroku Koho   1996-2006
Kokai Jitsuyo Shinan Koho    1971-2006   Toroku Jitsuyo Shinan Koho   1994-2006

Electronic data base consulted during the international search (name of data base and, where practicable, search terms used)
BIOSIS(STN), CAplus(STN), EMBASE(STN), MEDLINE(STN), REGISTRY(STN),
JSTPlus(JDream2)

C. DOCUMENTS CONSIDERED TO BE RELEVANT

| Category* | Citation of document, with indication, where appropriate, of the relevant passages | Relevant to claim No. |
|---|---|---|
| Y | UCHIKAWA, O. et al., "Synthesis of a novel series of tricyclic indan derivatives as melatonin receptor agonists", Journal of Medicinal Chemistry, 2002, Vol.45, No.12, pages 4222 to 4239 | 1-3,7-9 |
| Y A | Yasuo HISHIKAWA et al., 'Koreisha no Seitai Rhythm Ijo to Lifestyle ni Kansuru Kenkyu Alzheimer-gata Jakunen Chiho no Suimin· Kodo Shogai ni Taisuru Melatonin Hoju Ryoho no Kaihatsu ni Kansuru Kenkyu (Koseisho S)', Choju Kagaku Sogo Kenkyu, 1997, Vol.1996(3), pages 73 to 76 | 1,7 2-3,8-9 |

| ☒ Further documents are listed in the continuation of Box C. | | ☐ See patent family annex. |
|---|---|---|

| * | Special categories of cited documents: |
|---|---|
| "A" | document defining the general state of the art which is not considered   to be of particular relevance |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
|---|---|
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
|---|---|
| 08 May, 2006 (08.05.06) | 16 May, 2006 (16.05.06) |

| Name and mailing address of the ISA/ | Authorized officer |
|---|---|
| Japanese Patent Office | |
| Facsimile No. | Telephone No. |

Form PCT/ISA/210 (second sheet) (April 2005)

**INTERNATIONAL SEARCH REPORT**

International application No.

PCT/JP2006/307055

C (Continuation). DOCUMENTS CONSIDERED TO BE RELEVANT

| Category* | Citation of document, with indication, where appropriate, of the relevant passages | Relevant to claim No. |
|---|---|---|
| Y<br>A | JP 10-287665 A (Takeda Chemical Industries, Ltd.),<br>27 October, 1998 (27.10.98),<br>Particularly, Par. Nos. [0136], [0137]<br>& WO 1997/032871 A1    & EP 885210 B1<br>& US 6034239 A | 2-3,8-9<br>1,7 |
| Y<br>A | Toru YAMAGUCHI et al., "Kyo no Chiryo Hoshin 2004 Nenban (Volume 46)", Igaku-Shoin Ltd., 2004, pages 1576 to 1577 | 2-3,8-9<br>1,7 |

Form PCT/ISA/210 (continuation of second sheet) (April 2005)

**INTERNATIONAL SEARCH REPORT**

International application No.

PCT/JP2006/307055

Continuation of A. CLASSIFICATION OF SUBJECT MATTER
(International Patent Classification (IPC))

*A61P43/00* (2006.01)

(According to International Patent Classification (IPC) or to both national classification and IPC)

Form PCT/ISA/210 (extra sheet) (April 2005)

<table>
<tr><td>**INTERNATIONAL SEARCH REPORT**</td><td>International application No.<br>PCT/JP2006/307055</td></tr>
</table>

**Box No. II     Observations where certain claims were found unsearchable (Continuation of item 2 of first sheet)**

This international search report has not been established in respect of certain claims under Article 17(2)(a) for the following reasons:

1. [×]  Claims Nos.:  4-6
      because they relate to subject matter not required to be searched by this Authority, namely:
   The inventions as set forth in claims 4 to 6 pertain to methods for treatment of the human body by therapy.

2. [ ]  Claims Nos.:
      because they relate to parts of the international application that do not comply with the prescribed requirements to such an extent that no meaningful international search can be carried out, specifically:

3. [ ]  Claims Nos.:
      because they are dependent claims and are not drafted in accordance with the second and third sentences of Rule 6.4(a).

**Box No. III     Observations where unity of invention is lacking (Continuation of item 3 of first sheet)**

This International Searching Authority found multiple inventions in this international application, as follows:

1. [ ]  As all required additional search fees were timely paid by the applicant, this international search report covers all searchable claims.

2. [ ]  As all searchable claims could be searched without effort justifying an additional fee, this Authority did not invite payment of any additional fee.

3. [ ]  As only some of the required additional search fees were timely paid by the applicant, this international search report covers only those claims for which fees were paid, specifically claims Nos.:

4. [ ]  No required additional search fees were timely paid by the applicant.   Consequently, this international search report is restricted to the invention first mentioned in the claims; it is covered by claims Nos.:

**Remark on Protest**
the

[ ]  The additional search fees were accompanied by the applicant's protest and, where applicable, payment of a protest fee..

[ ]  The additional search fees were accompanied by the applicant's protest but the applicable protest fee was not paid within the time limit specified in the invitation.

[ ]  No protest accompanied the payment of additional search fees.

Form PCT/ISA/210 (continuation of first sheet (2)) (April 2005)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 6034239 A

**Non-patent literature cited in the description**

- **VITIELLO MV ; BORSON S.** Sleep disturbances in patients with Alzheimer's disease: epidemiology, pathophysiology and treatment. *CNS Drugs,* 2001, vol. 15, 777-96
- **BLIWISE DL.** Sleep disorders in Alzheimer's disease and other dementias. *Clin Cornerstone,* 2004, vol. 6, 16-28
- **LUBOSHITZKY R ; SHEN-ORR Z ; TZISCHICHIN-SKY O ; MALDONADO M ; HERER P ; LAVIE P.** Actigraphic sleep-wake patterns and urinary 6-sulfatoxymelatonin excretion in patients with Alzheimer's disease. *Chronobiol Int.,* 2001, vol. 18, 513-24
- **MISHIMA K ; TOZAWA T ; SATOH K ; MATSUMO-TO Y ; HISHIKAWA Y ; OKAWA M.** Melatonin secretion rhythm disorders in patients with senile dementia of Alzheimer's type with disturbed sleep-waking. *Biol Psychiatry,* 1999, vol. 45, 417-21